(19) Europäisches Patentamt
European Patent Office
Office européen des brevets

(11) Veröffentlichungsnummer: **0 068 283**
**B1**

(12) **EUROPÄISCHE PATENTSCHRIFT**

(45) Veröffentlichungstag der Patentschrift: **19.09.84**

(51) Int. Cl.³: **C 07 C 85/06,** C 07 C 87/50

(21) Anmeldenummer: **82105227.1**

(22) Anmeldetag: **15.06.82**

(54) **Verfahren zur Herstellung von alkylsubstituierten Anilinen.**

(30) Priorität: **27.06.81 DE 3125295**
**02.12.81 DE 3147734**

(43) Veröffentlichungstag der Anmeldung:
**05.01.83 Patentblatt 83/1**

(45) Bekanntmachung des Hinweises auf die Patenterteilung:
**19.09.84 Patentblatt 84/38**

(84) Benannte Vertragsstaaten:
**BE CH DE GB IT LI**

(56) Entgegenhaltungen:
**EP - A - 0 042 568**
**DE - A - 2 003 842**
**DE - A - 2 026 053**
**DE - A - 2 516 316**
**DE - A - 3 013 401**
**US - A - 3 272 865**

**CHEMICAL ABSTRACTS, Band 81, 1974, Seite 387, Zusammenfassung Nr. 135685v, Columbus, Ohio (US)**
**CHEMICAL ABSTRACTS, Band 81, 1974, Seite 387, Zusammenfassung Nr. 135688y, Columbus, Ohio (US)**

(73) Patentinhaber: **BAYER AG, Konzernverwaltung RP Patentabteilung, D-5090 Leverkusen 1 Bayerwerk (DE)**

(72) Erfinder: **Le Blanc, Helmut, Dr., Neuenhaus 26, D-5093 Burscheid (DE)**
Erfinder: **Wedemeyer, Karlfried, Dr., Bilharzstrasse 7, D-5000 Koeln 80 (DE)**

**EP 0 068 283 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von alkylsubstituierten Anilinen durch Umsetzung von alkylsubstituierten Phenolen in der Dampfphase mit Ammoniak in Gegenwart eines Aluminiumoxidkatalysators.

Es ist bekannt, Phenole in der Dampfphase mit Ammoniak unter Druck an Aluminiumoxid-haltigen Katalysatoren zu den entsprechenden Anilinen umzusetzen (US-PS 1 935 209, US-PS 2 013 873, US-PS 3 272 865, DE-AS 2 026 053, Chemical Abstracts 81, 1974, 387 Nr. 135685v und DE-OS 2 003 842). Obwohl in den genannten Druckschriften eine Vielzahl von Phenolen als geeignete Ausgangsverbindungen für die Umsetzung aufgezählt werden, werden in den dort angegebenen Beispielen nur einige wenige Phenole, insbesondere Phenol selbst, m- und p-Kresol und 3,5-Xylenol eingesetzt (US-PS 3 272 865, US-PS 1 935 209 und US-PS 2 013 873).

Der DE-OS 2 516 316 zufolge treten bei Aminierungsreaktionen mit substituierten Phenolen Schwierigkeiten auf. So wird beispielsweise erwähnt, daß bei Einsatz von m-Kresol bis zu 10% Nebenprodukte entstehen. Zur Vermeidung dieser Nachteile wird in der DE-OS 2 516 316 der Zusatz von Toluol empfohlen. Nach den Beispielen der DE-OS 2 516 316 setzt man pro Volumenteil Phenol 2 Volumenteile Toluol zu, das anschließend vom Produktgemisch destillativ abgetrennt, kondensiert und rückgeführt werden muß. Neben dem dazu erforderlichen apparativen Mehraufwand ist der Einsatz des leicht brennbaren Toluols bei den hohen Reaktionstemperaturen auch aus sicherheitstechnischen Gründen nicht unbedenklich.

Nach der japanischen Patentanmeldung 49-29 176 (1974) entsteht bei der Umsetzung von m-Kresol mit Ammoniak in der Gasphase m-Toluidin mit einer Selektivität von nur 81%.

Es wurde nun ein Verfahren zur Herstellung von alkylsubstituierten Anilinen durch Umsetzung von alkylsubstituierten Phenolen der Formel

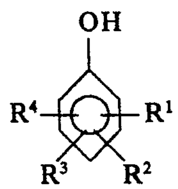

worin

$R^1$ bis $R^4$ gleich oder verschieden sind und für Wasserstoff oder geradkettige oder verzweigte oder cyclische Alkylreste stehen und wobei mindestens ein Rest geradkettiges oder verzweigtes oder cyclisches Alkyl bedeutet,

in der Dampfphase mit Ammoniak in Gegenwart eines Aluminiumoxid-Katalysators gefunden, das dadurch gekennzeichnet ist, daß man die dampfförmigen Ausgangsprodukte bei Temperaturen von 360 bis 460°C mit einer Strömungsgeschwindigkeit von mindestens 20 cm/h (bezogen auf flüssige Volumina) durch den Reaktor leitet und daß das Molverhältnis von Ammoniak zu alkylsubstituierten Phenolen mindestens 2 : 1 und der Druck mindestens 15 bar beträgt.

Als geradkettige oder verzweigte oder cyclische Alkylreste seien solche mit 1 bis 7 Kohlenstoffatomen, bevorzugt 1 bis 3 Kohlenstoffatomen, genannt, z. B. der Methyl-, Ethyl-, n-Propyl-, iso-Propyl-, n-Butyl-, iso-Butyl-, tert.-Butyl-, der Hexyl-, der Cyclohexyl- und der Methylcyclohexylrest, bevorzugt der Methylrest.

Beispielsweise werden als Ausgangsmaterial die isomeren Kresole und Xylenole, bevorzugt m-Kresol und 2,6-Xylenol, eingesetzt.

Nach oben ist die Strömungsgeschwindigkeit lediglich durch apparative Gegebenheiten, z. B. durch zu hohen Druckverlust oder zu starken Katalysatorabrieb, begrenzt. Bevorzugt leitet man die Ausgangsprodukte, bestehend aus $NH_3$ und alkylsubstituiertem Phenol, mit einer Strömungsgeschwindigkeit von 20 bis 3200 cm/h, besonders bevorzugt mit einer Strömungsgeschwindigkeit von 160 bis 1600 cm/h, über einen Verdampfer durch den Reaktor. Die Strömungsgeschwindigkeit v ist dabei wie folgt definiert:

$$v = \frac{\text{Durchsatz der flüssigen Volumina von } NH_3 \text{ und alkylsubst. Phenol}}{\text{Grundfläche des Reaktors} \cdot \text{Zeit}} \left[\frac{cm^3}{cm^2 \cdot h}\right] = \left[\frac{cm}{h}\right]$$

Die angegebenen Volumina für $NH_3$ und das alkylsubstituierte Phenol beziehen sich auf flüssiges $NH_3$ bei 40°C (d = 0,58) und flüssiges oder geschmolzenes Alkylphenol bei 60°C (d = 1,0).

Zur Erhöhung der Strömungsgeschwindigkeit kann man den Ausgangsprodukten inerte Gase, wie Stickstoff und/oder Wasserstoff und/oder Edelgase, wie Helium, Neon, Argon, Krypton und/oder Xenon, und/oder Dämpfe, wie Wasserdampf und/oder Dämpfe von unter den Reaktionsbedingungen

inerten organischen Verbindungen, wie Benzol, Toluol, Diphenyl und/oder Diphenylether, zumischen. Im allgemeinen gibt man 0,1 bis 50 Mol.-%, bevorzugt 2 bis 30 Mol.-%, bezogen auf das Ammoniak/Phenol-Gemisch, an inerten Gasen und/oder Dämpfen den Ausgangsprodukten zu.

Das erfindungsgemäße Verfahren wird üblicherweise bei einem Molverhältnis von Ammoniak zu Phenol von mindestens etwa 2 : 1 und einem Mindestdruck von etwa 15 bar durchgeführt. Nach oben bestehen sowohl beim Ammoniak/Alkylphenol-Molverhältnis als auch beim Druck lediglich technische und wirtschaftliche Grenzen. Bevorzugt wird das erfindungsgemäße Verfahren bei einem Molverhältnis von Ammoniak zu dem eingesetzten Alklphenol von 2 : 1 bis 140 : 1, besonders bevorzugt von 3 : 1 bis 75 : 1, und bei Drücken von 15 bis 250, besonders bevorzugt von 70 bis 220, bar durchgeführt.

Das erfindungsgemäße Verfahren wird im allgemeinen bei Temperaturen von etwa 360 bis 460° C, bevorzugt bei 380 bis 440° C, besonders bevorzugt bei 400 bis 430° C, durchgeführt.

Als Katalysatoren haben sich Aluminiumoxidkatalysatoren bewährt. Bevorzugt werden Aluminiumoxidkatalysatoren in das erfindungsgemäße Verfahren eingesetzt, die mindestens 95 Gew.-% Aluminiumoxid, weniger als 0,5% Alkalimetall (angegeben als Alkalimetalloxid) und weniger als 0,5% Eisen (angegeben als Eisenoxid) enthalten. Besonders bevorzugt wird ein hochreiner Aluminiumoxidkatalysator eingesetzt, der höchstens 0,2 Gew.-% Alkalimetall (angegeben als Alkalimetalloxid) und höchstens 0,3 Gew.-% Eisenmetall (angegeben als Eisenoxid) enthält.

Die einzusetzenden Aluminiumoxidkatalysatoren können in Form von Preßlingen oder Kugeln verwendet werden, bevorzugt werden Preßlinge mit einem Durchmesser von etwa 0,5 mm bis etwa 10 mm eingesetzt.

Das erfindungsgemäße Verfahren kann beispielsweise wie folgt durchgeführt werden:

Flüssiges Alkylphenol und flüssiges Ammoniak werden mit einer Strömungsgeschwindigkeit, die über 20 cm/h liegt, einem Verdampfer zugeführt, dort gemeinsam verdampft und dann durch einen mit dem Aluminiumoxidkatalysator gefüllten Röhrenreaktor unter Druck bei höheren Temperaturen geleitet. Das den Reaktor verlassende Reaktionsgemisch wird entspannt und kondensiert. Anschließend wird das Kondensat in an sich bekannter Weise fraktioniert destilliert. Zur Rückführung des nicht-umgesetzten Ammoniakanteils kann der Ammoniak bei ca. 50 bar von dem Produktgemisch abdestilliert und im Kreise gefahren werden.

Gemäß einer anderen Variante des erfindungsgemäßen Verfahrens können flüssiges Ammoniak und flüssiges Alkylphenol auch getrennt verdampft und erst vor Eintritt in den Reaktor gemischt werden.

Möglich ist auch, daß das Phenol von überhitztem $NH_3$ aufgeheizt und verdampft wird.

Zusätze wie Wasser und/oder inerte organische Lösungsmittel können gemeinsam mit dem $NH_3$/Phenol-Gemisch oder mit einem der beiden Reaktionspartner verdampft werden. Ebenso kann das Wasser und/oder das inerte organische Lösungsmittel getrennt verdampft und der Reaktionspartner erst vor Eintritt in den Reaktor zudosiert werden.

Das erfindungsgemäße Verfahren kann sowohl diskontinuierlich als auch kontinuierlich durchgeführt werden.

Mit Hilfe des erfindungsgemäßen Verfahrens können alkylsubstituierte Aniline in hoher Selektivität bei guten Raum/Zeit-Ausbeuten erhalten werden, wie die Versuchsbeispiele zeigen.

Überraschenderweise eignet sich das erfindungsgemäße Verfahren besonders zur Herstellung von 2,6-Xylidin aus 2,6-Xylenol, das praktisch frei von störenden isomeren 2,4- und 2,5-Xylidinen erhalten wird. Die Isolierung des 2,6-Xylidins in über 99%iger Reinheit ist deshalb ohne Schwierigkeiten gewährleistet.

Dies überrascht um so mehr, als 2,6-Xylidin bislang durch aufwendige Verfahren nach der DE-AS 1 933 636 und der DE-AS 2 208 827 hergestellt wurde und es unter den bekannten Aminierungsbedingungen nicht gelang, 2,6-Xylidin in guten Ausbeuten und frei von störenden Isomeren herzustellen. So liefert die Umsetzung von 2,6-Xylenol mit Ammoniak in der Gasphase unter den in der DE-AS 2 026 053 beschriebenen Aminierungsbedingungen nur eine unbefriedigende Ausbeute an 2,6-Xylidin. Außerdem verläuft die Reaktion mit einer schlechten Selektivität unter Bildung beträchtlicher Anteile von isomeren 2,4- und 2,5-Xylidinen, die bei der Aufarbeitung große Schwierigkeiten bereiten, da sich vor allem das 2,4-Dimethylanilin nicht oder nur mit sehr großem Destillationsaufwand abtrennen läßt.

2,6-Xylidin ist ein Zwischenprodukt zur Herstellung von Herbiziden (vgl. DE-OS 2 648 008, DE-OS 2 305 495, US-PS 3 952 056 und US-PS 4 019 894).

Das erfindungsgemäße Verfahren sei anhand der nachfolgenden Beispiele erläutert:

### Beispiel 1

Ein Reaktor von 76 cm Länge und 1 cm Durchmesser, der mit 60 ml Aluminiumoxidkatalysator gefüllt ist (Korngröße: 0,5 bis 1,0 mm), wird in einem Salzbad auf 400° C aufgeheizt.

In einem Vorverdampfer verdampft man ein Gemisch aus 39,3 ml flüssigem Ammoniak und 2,7 ml 2,6-Xylenol (Molverhältnis 60 : 1) in der Stunde und leitet das auf Reaktionstemperatur gebrachte Gasgemisch bei einem Druck von 190 bar durch den Reaktor. Die Ausgangsprodukte werden dabei mit

einer Strömungsgeschwindigkeit von 53,5 cm/h (bezogen auf flüssige Volumina) durch den Reaktor geleitet. Das entspannte Xylidin-Gemisch wird kondensiert und gaschromatographisch analysiert. Das Produkt setzt sich zusammen aus:

0,76% Anilin
1,87% o-Toluidin
1,41% p-Toluidin
93,2% 2,6-Xylidin
0,08% 2,4-Xylidin
0,13% 2,5-Xylidin
0,23% 2,6-Xylenol und
1,43% 2,4,6-Mesidin.

Beispiel 2 (Vergleichsbeispiel)

Die Arbeitsweise von Beispiel 1 wird mit der Änderung wiederholt, daß ein Gemisch aus 13,6 ml Ammoniak und 0,9 ml 2,6-Xylenol (Molverhältnis 60 : 1) pro Stunde verdampft und mit einer Strömungsgeschwindigkeit von 18,5 cm/h (bezogen auf flüssige Volumina) durch den Reaktor geleitet wird. Das Produktgemisch setzt sich zusammen aus:

0,74% Anilin
2,7% o-Toluidin
1,3% p-Toluidin
92,0% 2,6-Xylidin
0,14% 2,4-Xylidin
0,12% 2,5-Xylidin
0,18% 2,6-Xylenol und
2,4% 2,4,6-Mesidin.

Beispiel 3 (Vergleichsbeispiel)

Die Arbeitsweise von Beispiel 1 wird mit der Änderung wiederholt, daß ein Gemisch aus 4,2 ml Ammoniak und 0,3 ml 2,6-Xylenol (Molverhältnis 60 : 1) pro Stunde verdampft und mit einer Strömungsgeschwindigkeit von 5,7 cm/h (bezogen auf flüssige Volumina) durch den Reaktor geleitet wird. Das Produktgemisch setzt sich zusammen aus:

0,5% Anilin
3,2% o-Toluidin
1,06% p-Toluidin
91,4% 2,6-Xylidin
0,22% 2,4-Xylidin
0,13% 2,5-Xylidin
0,18% 2,6-Xylenol und
3,0% 2,4,6-Mesidin.

Beispiel 4

Die Arbeitsweise von Beispiel 1 wird mit der Änderung wiederholt, daß ein Gemisch aus 57,6 ml Ammoniak und 2,0 ml 2,6-Xylenol (Molverhältnis 120 : 1) pro Stunde verdampft und mit einer Strömungsgeschwindigkeit von 75,9 cm/h (bezogen auf flüssige Volumina) durch den Reaktor geleitet wird. Das Produkt setzt sich zusammen aus:

0,53% Anilin
1,56% o-Toluidin
1,17% p-Toluidin
92,8% 2,6-Xylidin
0,08% 2,4-Xylidin
0,12% 2,5-Xylidin
2,0% 2,6-Xylenol und
1,31% 2,4,6-Mesidin.

Beispiel 5 (Vergleichsbeispiel)

Die Arbeitsweise von Beispiel 1 wird mit der Änderung wiederholt, daß ein Gemisch aus 3,0 ml Ammoniak und 0,1 ml 2,6-Xylenol (Molverhältnis 120 : 1) pro Stunde verdampft und mit einer Strömungsgeschwindigkeit von 3,9 cm/h (bezogen auf flüssige Volumina) durch den Reaktor geleitet wird.
Das Produktgemisch setzt sich zusammen aus:

0,69% Anilin
4,0% o-Toluidin
1,18% p-Toluidin
90,0% 2,6-Xylidin
0,29% 2,4-Xylidin
0,11% 2,5-Xylidin
0,08% 2,6-Xylenol und
2,8% 2,4,6-Mesidin.

Beispiel 6

Die Arbeitsweise von Beispiel 1 wird mit der Änderung wiederholt, daß ein Gemisch aus 22,1 ml Ammoniak und 3,1 ml 2,6-Xylenol (Molverhältnis 30 : 1) pro Stunde verdampft und mit einer Strömungsgeschwindigkeit von 32,1 cm/h (bezogen auf flüssige Volumina) durch den Reaktor geleitet wird.
Das Produkt setzt sich zusammen aus:

0,8% Anilin
3,0% o-Toluidin
1,31% p-Toluidin
92,0% 2,6-Xylidin
0,22% 2,4-Xylidin
0,12% 2,5-Xylidin
0,33% 2,6-Xylenol und
1,75% 2,4,6-Mesidin.

Beispiel 7 (Vergleichsbeispiel)

Die Arbeitsweise von Beispiel 1 wird mit der Änderung wiederholt, daß ein Gemisch aus 8,9 ml Ammoniak und 1,2 ml 2,6-Xylenol (Molverhältnis 30 : 1) pro Stunde verdampft und mit einer Strömungsgeschwindigkeit von 12,9 cm/h (bezogen auf flüssige Volumina) durch den Reaktor geleitet wird.
Das Produktgemisch setzt sich zusammen aus:

0,82% Anilin
5,9% o-Toluidin
1,0% p-Toluidin
87,84% 2,6-Xylidin
0,57% 2,4-Xylidin
0,14% 2,5-Xylidin
0,47% 2,6-Xylenol und
2,76% 2,4,6-Mesidin.

Beispiel 8 (Zusatz von Wasser)

Ein Reaktor von 34 cm Länge und 1 cm Durchmesser, der mit 27 ml Aluminiumoxidkatalysator gefüllt ist (Korngröße 0,5 bis 1,0 mm) wird in einem Salzbad auf 400° C aufgeheizt.

In einem Vorverdampfer verdampft man ein Gemisch aus 15,3 ml flüssigem Ammoniak, 1,1 ml 2,6-Xylenol und 1,0 ml Wasser in der Stunde und leitet das 400° C heiße Gasgemisch bei einem Druck von 190 bar mit einer Strömungsgeschwindigkeit von 22,2 cm/h (bezogen auf flüssige Volumina) durch den Reaktor.

Das entspannte Xylidingemisch wird kondensiert und gaschromatographisch analysiert. Das Produkt setzt sich zusammen aus:

0,68% Anilin
2,6% o-Toluidin
0,7% p-Toluidin
91,8% 2,6-Xylidin
0,10% 2,4-Xylidin
0,02% 2,5-Xylidin
1,0% 2,6-Xylenol und
2,4% 2,4,6-Mesidin.

Beispiel 9

Ein Reaktor von 34 cm Länge und 1,0 cm Durchmesser, der mit 27 ml $Al_2O_3$-Katalysator gefüllt ist (Korngröße 0,5 bis 1,0 mm), wird in einem Salzbad auf 430° C aufgeheizt.
In einem Vorverdampfer verdampft man ein Gemisch aus 41,0 ml flüssigem $NH_3$ und 10,1 ml o-Kresol (Molverhältnis 15 : 1) in der Stunde und leitet das auf Reaktionstemperatur gebrachte Gasgemisch bei einem Druck von 190 bar durch den Reaktor. Die Ausgangsprodukte werden dabei mit einer Strömungsgeschwindigkeit von 65,0 cm/h (bezogen auf flüssige Volumina) durch den Reaktor geleitet. Das entspannte Toluidin-Gemisch wird kondensiert und gaschromatographisch analysiert.
Das Produkt setzt sich zusammen aus:

1,3% Anilin
96,7% o-Toluidin
0,26% m-Toluidin
0,61% 2,4-Xylidin
0,59% o-Kresol

Beispiel 10 (Vergleichsbeispiel)

Die Arbeitsweise von Beispiel 9 wird mit der Änderung wiederholt, daß ein Gemisch aus 4,7 ml $NH_3$ und 1,2 ml o-Kresol (Molverhältnis 15 : 1) pro Stunde verdampft und mit einer Strömungsgeschwindigkeit von 7,5 cm/h (bezogen auf flüssige Volumina) durch den Reaktor geleitet wird.
Das Produktgemisch setzt sich zusammen aus:

3,0% Anilin
92,68% o-Toluidin
0,12% p-Toluidin
1,23% m-Toluidin
1,76% 2,4-Xylidin
0,25% 2,5-Xylidin
0,62% o-Kresol

Beispiel 11

Die Arbeitsweise von Beispiel 9 wird mit der Änderung wiederholt, daß ein Gemisch aus 34 ml $NH_3$ und 8,4 ml p-Kresol (Molverhältnis 15 : 1) pro Stunde verdampft und mit einer Strömungsgeschwindigkeit von 54,0 cm/h (bezogen auf flüssige Volumina) durch den Reaktor geleitet wird.
Das Produktgemisch setzt sich zusammen aus:

0,31% Anilin
0,12% o-Toluidin
94,6% p-Toluidin
1,62% m-Toluidin
0,35% 2,4-Xylidin
0,42% p-Kresol
1,72% 4,4'-Dimethyl-diphenylamin

Beispiel 12 (Vergleichsbeispiel)

Die Arbeitsweise von Beispiel 9 wird mit der Änderung wiederholt, daß ein Gemisch aus 7,9 ml $NH_3$ und 2,0 ml p-Kresol (Molverhältnis 15 : 1) pro Stunde verdampft und mit einer Strömungsgeschwindigkeit von 12,7 cm/h (bezogen auf flüssige Volumina) durch den Reaktor geleitet wird.
Das Produktgemisch setzt sich zusammen aus:

| | |
|---|---|
| 6,7% | Anilin |
| 0,58% | o-Toluidin |
| 79,0% | p-Toluidin |
| 1,61% | m-Toluidin |
| 4,8% | 2,4-Xylidin |
| 0,49% | p-Kresol |
| 2,7% | 4,4'-Dimethyl-diphenylamin |

Beispiel 13

Die Arbeitsweise von Beispiel 9 wird mit der Änderung wiederholt, daß ein Gemisch aus 39,0 ml $NH_3$ und 9,6 ml m-Kresol (Molverhältnis 15 : 1) pro Stunde verdampft und mit einer Strömungsgeschwindigkeit von 61,8 cm/h (bezogen auf flüssige Volumina) durch den Reaktor geleitet wird.
Das Produktgemisch setzt sich zusammen aus:

| | |
|---|---|
| 98,5% | m-Toluidin |
| 0,14% | m-Kresol |
| 1,16% | 3,3'-Dimethyl-diphenylamin |

Beispiel 14 (Vergleichsbeispiel)

Die Arbeitsweise von Beispiel 9 wird mit der Änderung wiederholt, daß ein Gemisch aus 3,5 ml $NH_3$ und 0,9 ml m-Kresol (Molverhältnis 15 : 1) pro Stunde verdampft und mit einer Strömungsgeschwindigkeit von 5,6 cm/h (bezogen auf flüssige Volumina) durch den Reaktor geleitet wird.
Das Produktgemisch setzt sich zusammen aus:

| | |
|---|---|
| 90,0% | m-Toluidin |
| 0,49% | m-Kresol |
| 9,0% | 3,3'-Dimethyl-diphenylamin |

Beispiel 15

Die Arbeitsweise von Beispiel 1 wird mit der Änderung wiederholt, daß ein Gemisch aus 13,1 ml $NH_3$ und 4,1 ml o-Isopropylphenol (Molverhältnis 15 : 1) pro Stunde verdampft und mit einer Strömungsgeschwindigkeit von 21,8 cm/h (bezogen auf flüssige Volumina) durch den Reaktor geleitet wird.
Das Produktgemisch setzt sich zusammen aus:

| | |
|---|---|
| 8,4% | Anilin |
| 74,0% | o-Isopropyl-anilin |
| 11,8% | o-n-Propylanilin |
| 0,25% | m-Isopropyl-anilin |
| 0,35% | p-Isopropyl-anilin |
| 0,56% | o-Isopropyl-phenol |

Beispiel 16 (Vergleichsbeispiel)

Die Arbeitsweise von Beispiel 1 wird mit der Änderung wiederholt, daß ein Gemisch aus 8,5 ml $NH_3$ und 2,6 ml o-Isopropylphenol (Molverhältnis 15 : 1) pro Stunde verdampft und mit einer Strömungsgeschwindigkeit von 14,2 cm/h (bezogen auf flüssige Volumina) durch den Reaktor geleitet wird.

Das Produktgemisch setzt sich zusammen aus:

| | |
|---|---|
| 10,7% | Anilin |
| 69,3% | o-Isopropyl-anilin |
| 13,3% | o-n-Propylanilin |
| 0,28% | m-Isopropyl-anilin |
| 0,47% | p-Isopropyl-anilin |
| 0,63% | o-Isopropyl-phenol |

## Patentansprüche

1. Verfahren zur Herstellung von alkylsubstituierten Anilinen durch Umsetzung von alkylsubstituierten Phenolen der Formel

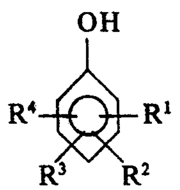

worin

$R^1$ bis $R^4$ gleich oder verschieden sind und für Wasserstoff oder geradkettige oder verzweigte oder cyclische Alkylreste stehen und wobei mindestens ein Rest geradkettiges oder verzweigtes oder cyclisches Alkyl bedeutet,

in der Dampfphase mit Ammoniak in Gegenwart eines Aluminiumoxid-Katalysators, dadurch gekennzeichnet, daß man die dampfförmigen Ausgangsprodukte bei Temperaturen von 360 bis 460°C mit einer Strömungsgeschwindigkeit von mindestens 20 cm/h (bezogen auf flüssige Volumina) durch den Reaktor leitet und daß das Molverhältnis von Ammoniak zu alkylsubstituierten Phenolen mindestens 2 : 1 und der Druck mindestens 15 bar beträgt.

2. Verfahren nach Anspruch 1, dadurch gekennzeichnet, daß man die dampfförmigen Ausgangsprodukte mit einer Strömungsgeschwindigkeit von 20 bis 3200 cm/h (bezogen auf flüssige Volumina) durch den Reaktor leitet.

3. Verfahren nach Anspruch 1, dadurch gekennzeichnet, daß man die dampfförmigen Ausgangsprodukte mit einer Strömungsgeschwindigkeit von 160 bis 160 cm/h (bezogen auf flüssige Volumina) durch den Reaktor leitet.

4. Verfahren nach Ansprüchen 1 bis 3, dadurch gekennzeichnet, daß man zur Erhöhung der Strömungsgeschwindigkeit den Ausgangsprodukten inerte Gase und/oder Dämpfe zumischt.

5. Verfahren nach Anspruch 4, dadurch gekennzeichnet, daß man als inerte Gase und/oder Dämpfe Stickstoff, Wasserstoff, Edelgase, Wasserdampf und/oder Dämpfe inerter organischer Verbindungen einsetzt.

6. Verfahren nach Ansprüchen 1 bis 5, dadurch gekennzeichnet, daß man den Ausgangsprodukten 0,1 bis 50 Mol.-%, bezogen auf das Ammoniak/Alkylphenol-Gemisch, an inerten Gasen und/oder Dämpfen zugibt.

7. Verfahren nach Ansprüchen 1 bis 5, dadurch gekennzeichnet, daß man den Ausgangsprodukten 2 bis 30 Mol.-%, bezogen auf das Ammoniak/Alkylphenol-Gemisch, an inerten Gasen und/oder Dämpfen zugibt.

## Claims

1. Process for the preparation of alkyl-substituted anilines by reacting alkyl-substituted phenols of the formula

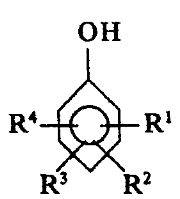

wherein

$R^1$ to $R^4$ are identical or different and represent hydrogen or straight-chain or branched or cyclic alkyl radicals, and wherein at least one radical denotes straight-chain or branched or cyclic alkyl,

in the vapour phase with ammonia in the presence of an aluminium oxide catalyst, characterised in that the starting materials in the vapour state are passed through the reactor at temperatures of 360 to 460° C and at a flow rate of at least 20 cm/h (relative to liquid volumes) and in that the molar ratio of ammonia to alkylsubstituted phenols is at least 2 : 1 and the pressure is at least 15 bar.

2. Process according to Claim 1, characterised in that the starting materials in the vapour state are passed through the reactor at a flow rate of 20 to 3,200 cm/h (relative to liquid volumes).

3. Process according to Claim 1, characterised in that the starting materials in the vapour state are passed through the reactor at a flow rate of 160 to 1,600 cm/h (relative to liquid volumes).

4. Process according to Claims 1 to 3, characterised in that inert gases and/or vapours are mixed with the starting materials in order to increase the flow rate.

5. Process according to Claim 4, characterised in that nitrogen, hydrogen, noble gases, water vapour and/or vapours of inert organic compounds are used as the inert gases and/or vapours.

6. Process according to Claims 1 to 5, characterised in that 0.1 to 50 mol %, relative to the ammonia/alkylphenol mixture, of inert gases and/or vapours are added to the starting materials.

7. Process according to Claims 1 to 5, characterised in that 2 to 30 mol %, based on the ammonia/alkylphenol mixture, of inert gases and/or vapours are added to the starting materials.

**Revendications**

1. Procédé de fabrication d'anilines alcoyl-substituées par réaction de phénols alcoyl-substitués de formule:

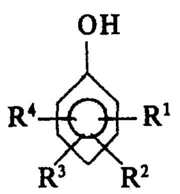

dans laquelle

$R^1$ et $R^4$ sont identiques ou différents et représentent de l'hydrogène ou des radicaux alcoyle à chaîne droite ou ramifiée ou cycliques, au moins un radical signifiant un alcoyle à chaîne droite ou ramifiée ou cyclique,

en phase gazeuse avec de l'ammoniac en présence d'un catalyseur d'oxyde d'aluminium, caractérisé en ce qu'on fait passer les produits de départ à l'état de vapeur à des températures de 360 à 460° C à une vitesse de circulation d'au moins 20 cm/h (par rapport aux volumes liquides) à travers le réacteur et en ce que le rapport molaire de l'ammoniac aux phénols alcoyl-substitués s'élève au moins à 2 : 1 et la pression au moins à 15 bars.

2. Procédé selon la revendication 1, caractérisé en ce qu'on fait passer les produits de départ à l'état de vapeur à une vitesse de circulation de 20 à 3200 cm/h (par rapport aux volumes liquides) à travers le réacteur.

3. Procédé selon la revendication 1, caractérisé en ce qu'on fait passer les produits de départ à l'état de vapeur à une vitesse de circulation de 160 à 1600 cm/h (par rapport aux volumes liquides) à travers le réacteur.

4. Procédé selon les revendications 1 à 3, caractérisé en ce que pour augmenter la vitesse de circulation des produits de départ onles mélange à des gaz et/ou des vapeurs inertes.

5. Procédé selon la revendication 4, caractérisé en ce qu'on utilise comme gaz et/ou vapeurs inertes de l'azote, de l'hydrogène, des gaz rares, de la vapeur d'eau et/ou des vapeurs de composés organiques inertes.

6. Procédé selon les revendications 1 à 5, caractérisé en ce qu'on ajoute aux produits de départ 0,1 à 50 moles %, par rapport au mélange ammoniac/alcoylphénol, de gaz et/ou de vapeurs inertes.

7. Procédé selon les revendications 1 à 5, caractérisé en ce qu'on ajoute aux produits de départ 2 à 30 moles %, par rapport au mélange ammoniac/alcoylphénol, de gaz et/ou de vapeurs inertes.